(19) Europäisches Patentamt
European Patent Office
Office européen des brevets

(11) **EP 4 043 883 A1**

(12) **EUROPÄISCHE PATENTANMELDUNG**

(43) Veröffentlichungstag:
**17.08.2022 Patentblatt 2022/33**

(21) Anmeldenummer: **21157263.1**

(22) Anmeldetag: **16.02.2021**

(51) Internationale Patentklassifikation (IPC):
**G01N 33/86** (2006.01) **G01N 21/82** (2006.01)
**G01N 33/49** (2006.01)

(52) Gemeinsame Patentklassifikation (CPC):
**G01N 33/86; G01N 33/4905;** G01N 2021/7783;
G01N 2800/224

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare Diagnostics Products GmbH**
**35041 Marburg (DE)**

(72) Erfinder:
• **Zander, Norbert**
**35039 Marburg (DE)**
• **Gerlach, Carina**
**35232 Dautphetal (DE)**
• **Gebauer, Regina**
**35466 Rabenau (DE)**
• **Timme, Michael**
**35091 Coelbe (DE)**

(54) **VERFAHREN ZUR BESTIMMUNG VON LUPUS ANTICOAGULANS IN EINER EINZIGEN GERINNUNGSREAKTION**

(57) Die Erfindung liegt auf dem Gebiet der Gerinnungsdiagnostik und betrifft ein Verfahren zur Detektion von Lupus anticoagulans.

**Beschreibung**

Die Erfindung liegt auf dem Gebiet der Gerinnungsdiagnostik und betrifft ein Verfahren zur Detektion von Lupus anticoagulans.

Lupus anticoagulans (LA) sind Immunglobuline und gehören zum Formenkreis der erworbenen Autoantikörper. Sie verursachen das Antiphospholipid-Syndrom (APS), eine der häufigsten Autoimmunerkrankungen, und rufen Thrombosen, wiederkehrende Fehlgeburten sowie Schwangerschaftskomplikationen hervor. Die Lupus anticoagulans Immunoglobuline sind sogenannte Antiphospholipid-Antikörper (APA), die an anionische Phospholipide, an Proteine oder an Protein/ Phospholipid-Komplexe binden. Die Antiphospholipid-Antikörper, die mit bestimmten Proteinen und Phospholipiden Komplexe bilden, stellen eine sehr heterogene Gruppe von Autoantikörpern dar, die gegen eine Vielzahl von Antigenen gerichtet sein können, wie z.B. gegen das Apolipoprotein β2-Glykoprotein I (β2GPI), Cardiolipin, Prothrombin, Protein C, Protein S, Thrombomodulin, Faktor XII und andere, sowie gegen Komplexe dieser Proteine mit Phospholipiden.

Die sogenannten Lupus Anticoagulanzien sind Antiphospholipid-Antikörper, die definitionsgemäß die Gerinnungszeiten von bestimmten Gerinnungstesten, z.B. von der APTT verlängern. Paradox ist, dass die Lupus Anticoagulanzien in vitro eine Hemmung der Gerinnungsreaktion bewirken, während in vivo eine erhöhte Gerinnungsreaktion (Hyperkoagulabilität) mit dem Antiphospholipid-Syndrom (APS) einhergeht.

Die Labordiagnose eines Antiphospholipid-Syndroms (APS) wird durch die Heterogenität der Antiphospholipid-Antikörper erschwert. Zum direkten Nachweis der Antikörper werden immunologische Verfahren angewandt. Hierbei werden allerdings auch viele Antikörper erfasst, die in vivo nicht prothrombotisch wirksam sind. Zum indirekten, funktionellen Nachweis der Antikörper werden Gerinnungsteste angewandt. Bei einem Gerinnungstest wird eine Plasmaprobe des Patienten typischerweise mit einem Gerinnungsaktivator, Phospholipiden und Calciumionen vermischt, und es wird die Zeit bis zur Gerinnselbildung gemessen. Besonders Teste, die auf der Bestimmung der DRVVT (Dilute Russell's Viper Venom Time) basieren, zeigen eine relativ gute Korrelation zur prothrombotischen Wirksamkeit der Antiphospholipid-Antikörper. Die Lupus-Diagnostik ist sehr aufwändig, weil jede Patientenprobe mehrere Untersuchungsschritte durchlaufen muss (zur Übersicht: Devreese, K. and Hoylaerts, M.F., Challenges in the diagnosis of the antiphospholipid syndrome. Clin. Chem. 2010, 56(6): 930-940).

Typischerweise wird eine Patientenprobe zur Diagnose eines Lupus anticoagulans mit zwei Varianten mindestens eines Gerinnungstestes untersucht. In der ersten, Lupus anticoagulans-sensitiven Variante wird der Test in Gegenwart einer relativ geringen Phospholipid-Konzentration durchgeführt (Screeningtest); in der zweiten, Lupus anticoagulans-insensitiven Variante wird derselbe Test in Gegenwart einer relativ hohen Phospholipid-Konzentration durchgeführt (Bestätigungstest). Eine gegenüber normalen Proben (die kein Lupus anticoagulans enthalten) verlängerte Gerinnungszeit in der ersten Testvariante (Screeningtest) in Kombination mit einer gegenüber normalen Proben normalen Gerinnungszeit in der zweiten Testvariante (Bestätigungstest) spricht für das Vorliegen von Lupus anticoagulans.

Ein besonders häufig eingesetzter Test ist der DRWT-Test, wobei die erste, Lupus anticoagulans-sensitive Variante mit einem Aktivierungsreagenz mit Russel-Viper-Gift und einer relativ geringen Phospholipid-Konzentration (LA1 Screeningreagenz) durchgeführt wird und die zweite, Lupus anticoagulans-insensitive Variante mit einem Aktivierungsreagenz mit Russel-Viper-Gift und einer hohen Phospholipid-Konzentration (LA2 Bestätigungsreagenz) durchgeführt wird. Ist der LA1-Test negativ, d.h. wird keine gegenüber dem Normal verlängerte Gerinnungszeit gemessen, ist die Durchführung des LA2-Testes nicht mehr notwendig. Ein anderer häufig eingesetzter Test ist der APTT-Test.

Nachteilig ist also, dass für die indirekte, funktionelle Detektion von Lupus anticoagulans immer mindestens zwei Gerinnungsteste durchgeführt werden müssen, was mit erhöhtem Material-, Zeit- und Arbeitsaufwand verbunden ist.

Der vorliegenden Erfindung liegt die Aufgabe zugrunde ein Verfahren zur indirekten, funktionellen Detektion von Lupus anticoagulans in einer Körperflüssigkeitsprobe bereit zu stellen, bei dem die vorgenannten Nachteile vermieden werden.

Es wurde gefunden, dass durch die Bestimmung der Gerinnungszeit und der Auswertung bestimmter Parameter der Reaktionskurve einer einzigen Gerinnungsreaktion, die mit einem Lupus anticoagulans-sensitiven APTT-Reagenz durchgeführt wird, die Detektion von Lupus anticoagulans möglich ist.

Gegenstand der vorliegenden Erfindung ist also ein Verfahren zur Detektion von Lupus anticoagulans in einer Plasmaprobe eines Patienten. Das Verfahren umfasst die folgenden Schritte:

- Bereitstellen eines Reaktionsgemisches durch Zugabe eines Lupus anticoagulans-sensitiven APTT-Reagenzes zu der Plasmaprobe und Starten der Gerinnungsreaktion,

- Messen einer Messgröße S des Reaktionsgemisches über die Zeit (t), resultierend in einer Funktion S(t) von zeitabhängigen Messwerten, und

- Bestimmen der Gerinnungszeit.

Zusätzlich werden erfindungsgemäß in dem Verfahren

a) die maximale Reaktionsgeschwindigkeit $v_{max}$ und/oder die maximale Reaktionsbeschleunigung $a_{max}$ der Funktion S(t) bestimmt, und

b) der Betrag der Differenz eines ersten Messwertes SB zu Beginn der Messung und eines zweiten Messwertes SE am Ende der Messung (|DeltaS|) ermittelt, und dann

c) die relative maximale Reaktionsgeschwindigkeit $v_{max\ rel}$ und/oder die relative maximale Reaktionsbeschleunigung $a_{max\ rel}$ mit folgenden Formeln bestimmt:

$$v_{max\ rel} = v_{max} / |DeltaS| \quad beziehungsweise$$
$$a_{max\ rel} = a_{max} / |DeltaS|.$$

Lupus anticoagulans wird schließlich detektiert, wenn die Gerinnungszeit gegenüber einem vorbestimmten Referenzwert verlängert ist und die relative maximale Reaktionsgeschwindigkeit $v_{max\ rel}$ und/oder die relative maximale Reaktionsbeschleunigung $a_{max\ rel}$ innerhalb eines vorbestimmten Lupus anticoagulans-spezifischen Wertebereiches liegen.

Die Plasmaprobe eines Patienten ist vorzugsweise eine plättchenarme Plasmaprobe eines Menschen. Vorzugsweise wird die plättchenarme Plasmaprobe aus Citratvollblut gewonnen.

Die APTT (aktivierte partielle Thromboplastinzeit) ist ein Test zur Kontrolle des intrinsischen Blutgerinnungssystems. Ein APTT-Reagenz, das einer zu testenden Probe zugegeben wird, enthält typischerweise Phospholipide ("partielle Thromboplastine"), eine oberflächenaktive Substanz (einen sogenannten "Kontaktaktivator"), wie z.B. Ellagsäure, Kaolin oder Silica, und optional Calciumionen. Ein Lupus anticoagulans-sensitives APTT-Reagenz enthält typischerweise eine im Vergleich zu einem Lupus anticoagulans-insensitiven APTT-Reagenz verringerte Konzentration an Phospholipiden, so dass eine Messung der Gerinnungszeit von Lupus anticoagulans-enthaltenden Proben mit einem solchen Reagenz zu einer gegenüber normalen (Lupus anticoagulans-freien) Proben verlängerten Gerinnungszeit führt. Lupus anticoagulans-sensitive APTT-Reagenzien können weitere Komponenten enthalten, wie z.B. bestimmte zweiwertige Metallionenproduzierende Substanzen, die den Effekt der Gerinnungszeitverlängernden Wirkung verstärken (siehe z.B. EP 3076178 A1). Die Calciumionen können alternativ in einem separaten Reagenz bereitgestellt werden, das der zu testenden Probe zusätzlich zu dem Lupus anticoagulans-sensitiven APTT-Reagenz zugegeben wird. Mit der Zugabe der Calciumionen wird die Gerinnungsreaktion im Reaktionsgemisch gestartet.

Das Messen einer Messgröße S des Reaktionsgemisches über die Zeit (t), resultierend in einer Funktion S(t) von zeitabhängigen Messwerten beginnt typischerweise mit der oder unmittelbar oder kurz nach Zugabe der Calciumionen.

Typische Messgrößen S des Reaktionsgemisches, die sich infolge der Gerinnungsreaktion über die Zeit ändern, sind z.B. die Trübung oder die Viskosität des Reaktionsgemisches infolge der Fibrinbildung im Reaktionsgemisch, die mit Hilfe optischer oder mechanischer Methoden quantitativ bestimmt werden können. Die kontinuierliche Bestimmung der Messgröße über einen gewissen Zeitraum resultiert in einer Funktion S(t) von zeitabhängigen Messwerten, also einer Reaktionskurve. Je nach Art der Messgröße kann sich eine Messgröße proportional oder antiproportional zur Gerinnungsreaktion ändern.

Die Bestimmung der Gerinnungszeit des Reaktionsgemisches kann mit jedem herkömmlichen Auswerteverfahren durchgeführt werden. Typischerweise wird unter dem Begriff "Gerinnungszeit" die Zeitspanne ab Start der Gerinnungsreaktion durch Zugabe der entsprechenden Reagenzien zur Probe bis zur fassbaren Bildung eines Fibringerinnsels in Sekunden verstanden. Die Gerinnungszeit wird vorzugsweise anhand der Reaktionskurve und einem geeigneten Auswerteverfahren bestimmt.

Die für die Probe bzw. das Reaktionsgemisch bestimmte Gerinnungszeit wird mit einem vorbestimmten Referenzwert, der eine normale Gerinnungszeit von einer verlängerten Gerinnungszeit unterscheidet, verglichen. Dieser Referenzwert wird vorab bestimmt, indem z.B. für eine statistisch aussagekräftige Anzahl von normalen (Lupus anticoagulans-freien) Plasmaproben und/oder für einen oder mehrere Normalplasmapools die Gerinnungszeit mit dem Lupus anticoagulans-sensitiven APTT-Reagenz bestimmt wird.

Die maximale Reaktionsgeschwindigkeit $v_{max}$ der Funktion S(t) kann bestimmt werden, indem -je nachdem ob sich die Messgröße proportional oder antiproportional zur Gerinnungsreaktion ändert- das Maximum oder das Minimum der ersten Ableitung (dS(t)/dt) der Funktion S(t) bestimmt wird.

Die maximale Reaktionsbeschleunigung $a_{max}$ der Funktion S(t) kann bestimmt werden, indem -je nachdem ob sich die Messgröße proportional oder antiproportional zur Gerinnungsreaktion ändert- das Maximum oder das Minimum der zweiten Ableitung ($d^2 S(t)/dt^2$) der Funktion S(t) bestimmt wird.

Ferner wird der Betrag der Differenz eines ersten Messwertes SB zu Beginn der Messung und eines zweiten Messwertes SE am Ende der Messung ($|DeltaS|$) ermittelt. Wie allgemein bekannt ist, verläuft eine Gerinnungsreaktion im Wesentlichen in drei Phasen. In der ersten Phase, zu Beginn der Messung, d.h. ab dem Zeitpunkt, in dem die Probe mit dem Gerinnungszeitreagenz und Calciumionen vermischt wurde und so die Gerinnungsreaktion gestartet wurde, ist über einen gewissen Zeitraum keine wesentliche Signaländerung, also keine Änderung der Messgröße S zu erkennen, d.h. die Reaktionskurve verläuft im Wesentlichen parallel zur X-Achse (t). In der darauffolgenden zweiten Phase ist eine Signaländerung zu erkennen, deren Änderungsgeschwindigkeit zunächst zunimmt und nach Erreichen eines Maximums wieder abnimmt. In der darauffolgenden dritten Phase, am Ende der Messung, hat die Signalhöhe ein Maximum erreicht, und es findet keine weitere Signaländerung mehr statt, d.h. die Reaktionskurve verläuft wieder parallel zur X-Achse (t), jedoch auf einem anderen Signalniveau als in der ersten Phase. Der erste Messwert SB ist also ein Messwert aus der ersten Phase der Gerinnungsreaktion; der zweite Messwert SE ist also ein Messwert aus der dritten Phase der Gerinnungsreaktion. Sowohl bei dem ersten Messwert SB als auch bei dem zweiten Messwert SE kann es sich um jeweils einen einzelnen Messwert oder um einen Mittelwert mehrerer (z.B. 2, 3, 4, 5 oder mehr) aufeinanderfolgender Messwerte innerhalb der jeweiligen Reaktionsphase handeln.

Ferner wird geprüft, ob die für die Probe bzw. das Reaktionsgemisch bestimmte relative maximale Reaktionsgeschwindigkeit $v_{max\ rel}$ und/oder die relative maximale Reaktionsbeschleunigung $a_{max\ rel}$ innerhalb eines vorbestimmten Lupus anticoagulans-spezifischen Wertebereiches liegen. Dieser Lupus anticoagulans-spezifische Wertebereich wird vorab bestimmt, indem z.B. in einer statistisch aussagekräftigen Anzahl von Lupus anticoagulans-enthaltenden Plasmaproben und von normalen (Lupus anticoagulans-freien) Plasmaproben (und/oder für einen oder mehrere Normalplasmapools) die Gerinnungsreaktion durch Zugabe des Lupus anticoagulans-sensitiven APTT-Reagenzes gestartet wird, eine Messgröße S des Reaktionsgemisches über die Zeit (t) gemessen wird, resultierend in einer Funktion S(t) von zeitabhängigen Messwerten und dann die relative maximale Reaktionsgeschwindigkeit $v_{max\ rel}$ und/oder die relative maximale Reaktionsbeschleunigung $a_{max\ rel}$ -wie oben in den Schritten a) bis c) beschrieben- bestimmt werden. Auf diese Weise kann ein Wertebereich bestimmt werden, der für Lupus anticoagulans-enthaltende Proben spezifisch ist.

Es wurde gefunden, dass mit dem erfindungsgemäßen Verfahren Lupus anticoagulans-enthaltende Proben von normalen Proben und von Proben mit anderen gerinnungszeitverlängernden Faktoren, wie z.B. Heparin, Gerinnungsfaktormängel, direkte orale Antikoagulanzien, zuverlässig differenziert werden können. Besonders vorteilhaft ist, dass die Auswertung einer einzigen Gerinnungsreaktion diese Differenzierung ermöglicht, so dass auf eine gemäß dem Stand der Technik notwendige zweite Gerinnungsreaktion mit einem anderen Reagenz verzichtet werden kann.

Ein weiterer Gegenstand der vorliegenden Erfindung ist ein automatisches Analysegerät, das so konfiguriert ist, dass es das vorbeschriebene erfindungsgemäße Verfahren durchführt.

Bekannte automatische Analysegeräte, die für die automatische Abarbeitung und Auswertung von Gerinnungstesten vorgesehen sind, umfassen mindestens (i) eine oder mehrere Pipettiervorrichtungen zum Transfer eines Probenvolumens und mindestens eines Reagenzvolumens in ein Reaktionsgefäß zur Herstellung eines Reaktionsgemisches, (ii) eine Messvorrichtung zum Messen einer Messgröße S des Reaktionsgemisches in dem Reaktionsgefäß über die Zeit (t), (iii) einen Datenspeicher zur Speicherung einer Funktion S(t) von zeitabhängigen Messwerten, die für eine Probe bzw. ein Reaktionsgemisch gemessen wurden, und

(iv) eine Auswerteeinrichtung, die so konfiguriert ist, dass sie die Funktion S(t) von zeitabhängigen Messwerten aus dem Datenspeicher zur Berechnung einer Gerinnungszeit verwendet.

Ein erfindungsgemäßes automatisches Analysegerät zeichnet sich dadurch aus, dass die Auswerteeinrichtung, zusätzlich so konfiguriert ist, dass sie

a) die maximale Reaktionsgeschwindigkeit $v_{max}$ und/oder die maximale Reaktionsbeschleunigung $a_{max}$ der Funktion S(t) bestimmt, und

b) den Betrag der Differenz eines ersten Messwertes SB zu Beginn der Messung und eines zweiten Messwertes SE am Ende der Messung ($|DeltaS|$) ermittelt, und dann

c) die relative maximale Reaktionsgeschwindigkeit $v_{max\ rel}$ und/oder die relative maximale Reaktionsbeschleunigung $a_{max\ rel}$ mit folgenden Formeln bestimmt:

$$v_{max\ rel} = v_{max} / |DeltaS| \quad \text{beziehungsweise}$$

$$a_{max\ rel} = a_{max} / |DeltaS|,$$

und

d) die bestimmte Gerinnungszeit mit einem vorbestimmten Referenzwert vergleicht, und

e) die relative maximale Reaktionsgeschwindigkeit $v_{max\ rel}$ und/oder die relative maximale Reaktionsbeschleunigung $a_{max\ rel}$ mit einem jeweils vorbestimmten Lupus anticoagulans-spezifischen Wertebereich vergleicht, und

f) als Ergebnis die Anwesenheit von Lupus anticoagulans in der Probe ausgibt, wenn die Gerinnungszeit gegenüber dem vorbestimmten Referenzwert verlängert ist und die relative maximale Reaktionsgeschwindigkeit $v_{max\ rel}$ und/oder die relative maximale Reaktionsbeschleunigung $a_{max\ rel}$ innerhalb des jeweiligen vorbestimmten Lupus anticoagulans-spezifischen Wertebereiches liegen.

Die Ausgabe des Ergebnisses der Anwesenheit von Lupus anticoagulans in der Probe erfolgt vorzugsweise an ein Anzeigemedium, beispielsweise einen Monitor, ein mobiles Endgerät oder einen Drucker, mit dem das Ergebnis einem Benutzer übermittelt werden kann.

Bei der Messvorrichtung zum Messen einer Messgröße S des Reaktionsgemisches kann es sich um eine Vorrichtung zum Messen einer optischen Eigenschaft, wie z.B. der Absorption, handeln, z.B. um ein Photometer.

## FIGURENBESCHREIBUNG

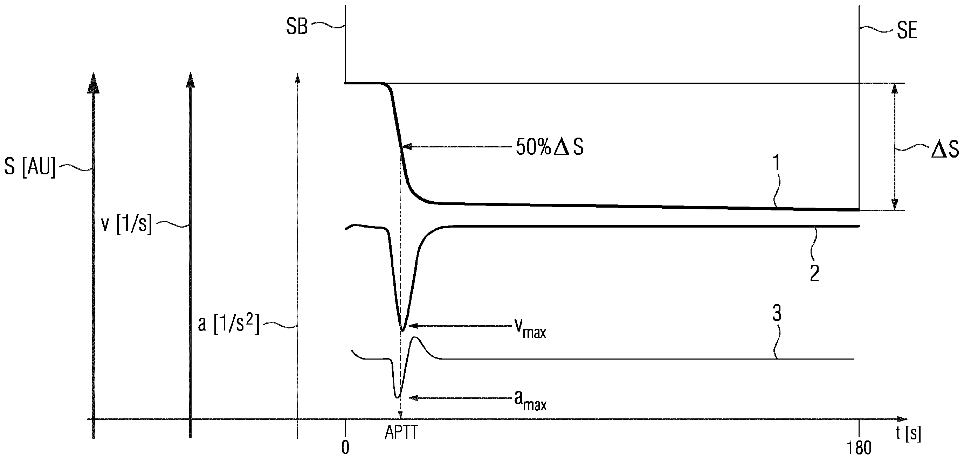

FIG. 1 zeigt eine typische APTT-Gerinnungskurve auf einem Sysmex CS-2100 Analysegerät. Kurve 1 zeigt zeitabhängig die optische Durchlässigkeit/Transmission (S) des Reaktionsgemischs in artifiziellen Einheiten [AU] über eine Zeit von 180 s. Die Differenz Delta S ($\Delta$S) der Durchlässigkeiten am Ende der Messung (Messwert SE zum Zeitpunkt t = 180 s) und zu Beginn der Messung (Messwert SB zum Zeitpunkt t = 0 s) kann in der Software ausgelesen werden. Die APTT-Gerinnungszeit wird bestimmt, indem der Zeitpunkt bestimmt wird, in dem der Messwert 50 % der Differenz Delta S ($\Delta$S) entspricht. Kurve 2 zeigt die numerische 1. Ableitung nach der Zeit (dS/dt), die ein Maß für die Geschwindigkeit v der Reaktion ist. Sie weist ein Minimum bei der höchsten Reaktionsgeschwindigkeit $v_{max}$ auf. Kurve 3 zeigt die numerische 2. Ableitung nach der Zeit ($d^2S/dt^2$), die ein Maß für die Beschleunigung a der Reaktion ist. Sie weist ein Minimum bei der höchsten Reaktionsbeschleunigung $a_{max}$ auf. Die numerischen Werte für die maximale Reaktionsgeschwindigkeit $v_{max}$ und die maximale Reaktionsbeschleunigung $a_{max}$ können über die Software ausgelesen werden. Die numerischen Werte für die relative Maximalgeschwindigkeit $v_{max\ rel}$ und die relative Maximalbeschleunigung $a_{max\ rel}$ ergeben sich durch Division der Absolutwerte für Maximalgeschwindigkeit und Maximalbeschleunigung durch den Betrag von Delta S.

Die Erfindung wird im Folgenden anhand eines Ausführungsbeispiels erläutert.

**BEISPIEL: Erfindungsgemäße Identifizierung Lupus antikoagulans-positiver Proben in jeweils einem einzigen APTT-Gerinnungsansatz**

Es wurden folgende Typen plättchenarmer Plasmaproben getestet:

| Probentyp | Beschreibung |
| --- | --- |
| Normal | Pool von > 100 Spenden Gesunder, stabilisiert; |
| Heparin | Pool von > 100 Spenden Gesunder, stabilisiert und versetzt mit unfraktioniertem oder niedermolekularem Heparin (UF bzw. LMW Heparin) ; |
| Faktor VIII-Mangel | Mischung von Faktor VIII-Mangelplasma und Normalpool; |
| Lupus anticoagulans (LA) | Einzelspenderplasmen, Lupus anticoagulans-positiv in einem auf dilute Rüssel's Viper Venom (DRVVT)-Test beruhenden Testsystem. |

Für die Messung der APTT wird das am Sysmex CS-2100 Analysegerät (Sysmex Corp.) zugelassene Testsetting verwendet. 50 μL Probe werden mit 50 μL Lupus anticoagulans sensitivem APTT-Reagenz (Actin FSL Reagenz, Siemens Healthcare Diagnostics Products GmbH) gemischt, das Phospholipide und Ellagsäure als Aktivator enthält. Nach 180 s Inkubation bei +37 °C werden zum Starten der Reaktion 50 μL 25 mM $CaCl_2$ zugefügt und die Messwerterfassung wird gestartet.

Die APTT-Gerinnungszeit wird bestimmt, indem der Zeitpunkt ermittelt wird, bei dem die gemessene optische Durchlässigkeit des Reaktionsgemisches 50 % der Differenz Delta S der Durchlässigkeiten am Ende der Messung (Messwert SE zum Zeitpunkt t = 180 s) und zu Beginn der Messung (Messwert SB zum Zeitpunkt t = 0 s) beträgt. Als verlängert gelten Gerinnungszeitergebnisse von mehr als 30 s.

Die relative maximale Reaktionsgeschwindigkeit $v_{max\ rel}$ wird bestimmt, indem zunächst die maximale Reaktionsgeschwindigkeit $v_{max}$ durch die Bestimmung des Minimums der 1. Ableitung der Reaktionskinetik nach der Zeit (dS/dt) ermittelt wird und $v_{max}$ schließlich durch den Betrag von Delta S dividiert wird.

Die relative maximale Reaktionsbeschleunigung $a_{max\ rel}$ wird bestimmt, indem zunächst die maximale Reakti-

onsbeschleunigung $a_{max}$ durch die Bestimmung des Minimums der 2. Ableitung der Reaktionskinetik nach der Zeit ($d^2S/dt^2$) ermittelt wird und $a_{max}$ schließlich durch den Betrag von Delta S dividiert wird.

Die Ergebnisse für jede Probe sind in Tabelle 1 gezeigt.

**Tabelle 1**

| Probe | APTT [s] | $v_{max\ rel}$ $[10^{-3}s^{-1}]$ | $a_{max\ rel}$ $[10^{-5}s^{-2}]$ |
|---|---|---|---|
| Normal 1 | 24,8 | 7,0 | 11,3 |
| Normal 2 | 27,3 | 6,9 | 11,1 |
| LMW Heparin 1,0 IU/mL | 50,4 | 3,9 | 5,2 |
| UF Heparin 0,6 IU/mL | 75,2 | 2,7 | 2,7 |
| Faktor VIII-Mangel 5 % - 1 | 46,7 | 2,7 | 2,8 |
| Faktor VIII-Mangel 5 % - 2 | 43,2 | 3,7 | 4,6 |
| LA 1 | 51,6 | 1,3 | 1,5 |
| LA 2 | 40,9 | 1,7 | 2,3 |
| LA 3 | 37,4 | 1,2 | 1,6 |
| LA 4 | 36,7 | 1,2 | 1,7 |
| LA 5 | 31,7 | 1,1 | 1,6 |
| LA 6 | 35,8 | 1,5 | 2,2 |
| LA 7 | 36,7 | 1,2 | 1,5 |
| LA 8 | 80,2 | 2,3 | 2,3 |
| LA 9 | 69,1 | 1,9 | 2,0 |
| LA 10 | 51,9 | 1,1 | 1,3 |

Erwartungsgemäß zeigen Heparinproben, Faktor VIII-Mangelproben (mit Mangel eines Faktors des intrinsischen Systems) sowie Lupus anticoagulans-positive Proben verlängerte Gerinnungszeiten (>30 s), während Normalproben keine verlängerten Gerinnungszeiten zeigen. Als Screeningtest für Lupus antikoagulans weist die APTT in diesem Experiment eine Sensitivität von 100 % auf.

Für Lupus anticoagulans-positive Proben zeigt sich ferner, dass der oben beschriebene Parameter $v_{max\ rel}$ in einem Wertebereich von 1.1 bis 2.3 $10^{-3}s^{-1}$ liegt, der für Lupus anticoagulans-positive Proben spezifisch ist. Ferner zeigt sich für Lupus anticoagulans-positive Proben, dass der ebenfalls oben beschriebene Parameter $a_{max\ rel}$ in einem Wertebereich von 1.3 bis 2.3 $10^{-5}s^{-2}$, liegt, der ebenfalls für Lupus anticoagulans-positive Proben spezifisch ist. Andere Proben (Normalproben, Heparinproben, Faktor VIII-Mangelproben) weisen jeweils größere numerische Werte für die Parameter $v_{max\ rel}$ und $a_{max\ rel}$ auf, die außerhalb der Lupus anticoagulans-spezifischen Wertebereiche für $v_{max\ rel}$ bzw. $a_{max\ rel}$ liegen.

Als Nachweis für Lupus anticoagulans eignet sich demnach ein zweistufiges Verfahren, das auf nur eine einzige APTT-Messung zurückgeht und die Gerinnungszeitbestimmung und die Bestimmung der Parameter $v_{max\ rel}$ und/oder $a_{max\ rel}$ umfasst.

Im vorliegenden Experiment weist das Verfahren für die Detektion von Lupus anticoagulans eine Sensitivität und eine Spezifität von jeweils 100 % auf.

## Patentansprüche

1. Verfahren zur Detektion von Lupus anticoagulans in einer Plasmaprobe eines Patienten, das Verfahren umfassend die Schritte:

   • Bereitstellen eines Reaktionsgemisches durch Zugabe eines Lupus anticoagulans-sensitiven APTT-Reagenzes zu der Plasmaprobe und Starten der Gerinnungsreaktion,
   • Messen einer Messgröße S des Reaktionsgemisches über die Zeit, resultierend in einer Funktion S(t) von zeitabhängigen Messwerten, und
   • Bestimmen der Gerinnungszeit,

   **dadurch gekennzeichnet, dass** zusätzlich

   a) die maximale Reaktionsgeschwindigkeit $v_{max}$ und/oder die maximale Reaktionsbeschleunigung $a_{max}$ der

Funktion S(t) bestimmt werden, und

b) der Betrag der Differenz eines ersten Messwertes SB zu Beginn der Messung und eines zweiten Messwertes SE am Ende der Messung (|DeltaS|) ermittelt wird, und dann

c) die relative maximale Reaktionsgeschwindigkeit $v_{max\,rel}$ und/oder die relative maximale Reaktionsbeschleunigung $a_{max\,rel}$ mit folgenden Formeln bestimmt werden:

$$v_{max\;rel} = v_{max} \;/\; |DeltaS| \quad beziehungsweise$$

$$a_{max\;rel} = a_{max} \;/\; |DeltaS|,$$

und

Lupus anticoagulans detektiert wird, wenn die Gerinnungszeit gegenüber einem vorbestimmten Referenzwert verlängert ist und die relative maximale Reaktionsgeschwindigkeit $v_{max\,rel}$ und/oder die relative maximale Reaktionsbeschleunigung $a_{max\,rel}$ innerhalb eines vorbestimmten Lupus anticoagulans-spezifischen Wertebereiches liegen.

2. Verfahren gemäß Anspruch 1, wobei das Lupus anticoagulans-sensitive APTT-Reagenz Phospholipide, einen Kontaktaktivator und optional Calciumionen enthält.

3. Verfahren gemäß Anspruch 1, wobei die maximale Reaktionsgeschwindigkeit $v_{max}$ dem Maximum oder dem Minimum der ersten Ableitung der Funktion S(t) der zeitabhängigen Messwerte entspricht.

4. Verfahren gemäß Anspruch 1, wobei die maximale Reaktionsbeschleunigung $a_{max}$ dem Maximum oder dem Minimum der zweiten Ableitung der Funktion S(t) der zeitabhängigen Messwerte entspricht.

5. Automatisches Analysegerät umfassend

(i) eine oder mehrere Pipettiervorrichtungen zum Transfer eines Probenvolumens und mindestens eines Reagenzvolumens in ein Reaktionsgefäß zur Herstellung eines Reaktionsgemisches,

(ii) eine Messvorrichtung zum Messen einer Messgröße S eines Reaktionsgemisches in dem Reaktionsgefäß über die Zeit (t),

(iii) einen Datenspeicher zur Speicherung einer Funktion S(t) von zeitabhängigen Messwerten, die für eine Probe bzw. ein Reaktionsgemisch gemessen wurden, und

(iv) eine Auswerteeinrichtung, die so konfiguriert ist, dass sie die Funktion S(t) von zeitabhängigen Messwerten aus dem Datenspeicher zur Berechnung einer Gerinnungszeit verwendet,

**dadurch gekennzeichnet, dass**
die Auswerteeinrichtung, zusätzlich so konfiguriert ist, dass sie

a) die maximale Reaktionsgeschwindigkeit $v_{max}$ und/oder die maximale Reaktionsbeschleunigung $a_{max}$ der Funktion S(t) bestimmt, und

b) den Betrag der Differenz eines ersten Messwertes SB zu Beginn der Messung und eines zweiten Messwertes SE am Ende der Messung (|DeltaS|) ermittelt, und dann

c) die relative maximale Reaktionsgeschwindigkeit $v_{max\,rel}$ und/oder die relative maximale Reaktionsbeschleunigung $a_{max\,rel}$ mit folgenden Formeln bestimmt:

$$v_{max\;rel} = v_{max} \;/\; |DeltaS| \quad beziehungsweise$$

$$a_{max\;rel} = a_{max} \;/\; |DeltaS|,$$

und

d) die bestimmte Gerinnungszeit mit einem vorbestimmten Referenzwert vergleicht, und

e) die relative maximale Reaktionsgeschwindigkeit $v_{max\,rel}$ und/oder die relative maximale Reaktionsbeschleunigung $a_{max\,rel}$ mit einem jeweils vorbestimmten Lupus anticoagulans-spezifischen Wertebereich vergleicht, und

f) als Ergebnis die Anwesenheit von Lupus anticoagulans in der Probe ausgibt, wenn die Gerinnungszeit gegenüber dem vorbestimmten Referenzwert verlängert ist und die relative maximale Reaktionsgeschwindigkeit $v_{max\,rel}$ und/oder die relative maximale Reaktionsbeschleunigung $a_{max\,rel}$ innerhalb des jeweiligen vorbestimmten Lupus anticoagulans-spezifischen Wertebereiches liegen.

6. Automatisches Analysegerät gemäß Anspruch 5, bei dem die Messvorrichtung zum Messen einer optischen Messgröße S eines Reaktionsgemisches geeignet ist.

7. Automatisches Analysegerät gemäß Anspruch 6, bei dem die Messvorrichtung zum Messen einer optischen Messgröße S eines Reaktionsgemisches ein Photometer ist.

Europäisches Patentamt
European Patent Office
Office européen des brevets

# EUROPÄISCHER RECHERCHENBERICHT

Nummer der Anmeldung

EP 21 15 7263

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2016/178651 A1 (SHIMA MIDORI [JP] ET AL) 23. Juni 2016 (2016-06-23) * Beispiel 1; Abb. 10A bis 10C; Par. 0004, 0056-0062, 0063-0119 * ----- | 1-7 | INV. G01N33/86 G01N21/82 G01N33/49 |
| Y | US 2016/291042 A1 (KUMANO OSAMU [JP] ET AL) 6. Oktober 2016 (2016-10-06) * Beispiel 1; Abb.12A bis 12C; Par. 0065-0071 * ----- | 1-4 | |
| X | SHIMONISHI NARUTO ET AL: "A Novel Assessment of Factor VIII Activity by Template Matching Utilizing Weighted Average Parameters from Comprehensive Clot Waveform Analysis", THROMBOSIS AND HAEMOSTASIS, Bd. 121, Nr. 02, 22. August 2020 (2020-08-22), Seiten 164-173, XP055823241, DE ISSN: 0340-6245, DOI: 10.1055/s-0040-1715838 Gefunden im Internet: URL:https://www.thieme-connect.com/products/ejournals/pdf/10.1055/s-0040-1715838.pdf > * Zusammenfassung; S. 165, Par.: Modified Clot Waveform Analysis * ----- -/-- | 1-4 | |

**RECHERCHIERTE SACHGEBIETE (IPC)**

G01N

Der vorliegende Recherchenbericht wurde für alle Patentansprüche erstellt

| Recherchenort | Abschlußdatum der Recherche | Prüfer |
|---|---|---|
| München | 13. Juli 2021 | Schalich, Juliane |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................................
& : Mitglied der gleichen Patentfamilie, übereinstimmendes Dokument

EPO FORM 1503 03.82 (P04C03)

Seite 1 von 3

**Europäisches Patentamt**

**European Patent Office**

**Office européen des brevets**

## EUROPÄISCHER RECHERCHENBERICHT

Nummer der Anmeldung

EP 21 15 7263

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | NOGAMI K. ET AL: "Modified clot waveform analysis to measure plasma coagulation potential in the presence of the anti-factor IXa/factor X bispecific antibody emicizumab", JOURNAL OF THROMBOSIS AND HAEMOSTASIS, Bd. 16, Nr. 6, 13. Mai 2018 (2018-05-13), Seiten 1078-1088, XP055822685, GB ISSN: 1538-7933, DOI: 10.1111/jth.14022 Gefunden im Internet: URL:https://onlinelibrary.wiley.com/doi/full-xml/10.1111/jth.14022> * das ganze Dokument * | 1-7 | |
| Y | US 2003/104493 A1 (ORTEL THOMAS L [US] ET AL) 5. Juni 2003 (2003-06-05) * Par.: 0006; Par.: 0015 * | 1-7 | |
| A | KUMANO OSAMU ET AL: "Lupus anticoagulant mixing tests for multiple reagents are more sensitive if interpreted with a mixing test-specific cut-off than index of circulating anticoagulant", RESEARCH AND PRACTICE IN THROMBOSIS AND HAEMOSTASIS, Bd. 2, Nr. 1, 1. Januar 2018 (2018-01-01), Seiten 105-113, XP055823219, GB ISSN: 2475-0379, DOI: 10.1002/rth2.12069 Gefunden im Internet: URL:https://www.ncbi.nlm.nih.gov/pmc/articles/PMC6055558/pdf/RTH2-2-105.pdf> * Table 1 * | 1-7 | RECHERCHIERTE SACHGEBIETE (IPC) |

-/--

Der vorliegende Recherchenbericht wurde für alle Patentansprüche erstellt

| Recherchenort | Abschlußdatum der Recherche | Prüfer |
|---|---|---|
| München | 13. Juli 2021 | Schalich, Juliane |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Europäisches
Patentamt

European
Patent Office

Office européen
des brevets

## EUROPÄISCHER RECHERCHENBERICHT

**Nummer der Anmeldung**

EP 21 15 7263

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | Anonymous: "Coagpia(TM)APTT-N", , 1. April 2011 (2011-04-01), Seiten 1-2, XP055823530, Gefunden im Internet: URL:https://manualzz.com/doc/6930596/coagpia%E2%84%A2aptt-n [gefunden am 2021-07-12] * das ganze Dokument * ----- | 1-7 | |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| München | 13. Juli 2021 | Schalich, Juliane |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
..............................................................................
& : Mitglied der gleichen Patentfamilie, übereinstimmendes
Dokument

EPO FORM 1503 03.82 (P04C03)

Seite 3 von 3

**ANHANG ZUM EUROPÄISCHEN RECHERCHENBERICHT
ÜBER DIE EUROPÄISCHE PATENTANMELDUNG NR.**

EP 21 15 7263

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-07-2021

| Im Recherchenbericht angeführtes Patentdokument | Datum der Veröffentlichung | Mitglied(er) der Patentfamilie | Datum der Veröffentlichung |
|---|---|---|---|
| US 2016178651 A1 | 23-06-2016 | JP 6430809 B2<br>JP 2016118442 A<br>US 2016178651 A1 | 28-11-2018<br>30-06-2016<br>23-06-2016 |
| US 2016291042 A1 | 06-10-2016 | CN 106018282 A<br>EP 3076173 A1<br>JP 6871673 B2<br>JP 2016194426 A<br>US 2016291042 A1 | 12-10-2016<br>05-10-2016<br>12-05-2021<br>17-11-2016<br>06-10-2016 |
| US 2003104493 A1 | 05-06-2003 | AU 2002367824 A1<br>EP 1436628 A2<br>JP 2005520170 A<br>US 2003104493 A1<br>WO 03083490 A2 | 13-10-2003<br>14-07-2004<br>07-07-2005<br>05-06-2003<br>09-10-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3076178 A1

**In der Beschreibung aufgeführte Nicht-Patentliteratur**

- **DEVREESE, K. ; HOYLAERTS, M.F.** Challenges in the diagnosis of the antiphospholipid syndrome. *Clin. Chem.,* 2010, vol. 56 (6), 930-940